# EUROPÄISCHE PATENTSCHRIFT

(11) **EP 1 838 239 B1**
(45) Veröffentlichungstag und Bekanntmachung des Hinweises auf die Patenterteilung: **13.03.2013**
(21) Anmeldenummer: 06706273.7
(22) Anmeldetag: 18.01.2006
(51) Int. Cl.: A61F 2/00

(54) **TESTEINHEIT FÜR EINMALUNTERSUCHUNGEN EINER KÖRPERFLÜSSIGKEIT**
TESTING UNIT FOR CARRYING OUT A ONE-TIME TESTING OF A BODY FLUID
UNITE D'ANALYSE A USAGE UNIQUE SERVANT A EFFECTUER DES ANALYSES D'UN LIQUIDE ORGANIQUE

(30) Priorität: 19.01.2005 DE 102005003789
(43) Veröffentlichungstag der Anmeldung: 03.10.2007
(73) Patentinhaber: F. Hoffmann-La Roche AG, 4070 Basel (CH); Roche Diagnostics GmbH, 68305 Mannheim (DE)
(72) Erfinder: ARNITZ, Theo, 68753 Waghäusel (DE)
(74) Vertreter: Pfiz, Thomas
(86) Internationale Anmeldenummer: PCT/EP2006/000385
(87) Internationale Veröffentlichungsnummer: WO 2006/077080

(56) Entgegenhaltungen:
- EP-A- 1 491 143
- WO-A-2004/091403
- WO-A-2005/001418
- DE-A1- 10 142 232
- DE-A1- 10 258 016
- US-A- 2 208 606
- US-A- 2 217 602
- US-A- 5 374 250
- US-A1- 2002 087 056
- US-A1- 2002 103 499

## Beschreibung

Die Erfindung betrifft eine Testeinheit für Einmaluntersuchungen einer Körperflüssigkeit, insbesondere Blut, mit einem in ein Körperteil einstechbaren Stechelement, das einen von einem Stechorgan zu einer Zielstelle führenden Kapillarkanal für den Transport der Körperflüssigkeit aufweist.

Testeinheiten dieser Art speziell für patientennahe Glukose-Meßsysteme dienen zur Selbstkontrolle der aktuellen Blutzuckerwerte eines Benutzers. In diesem Zusammenhang wurden bereits integrierte Systeme vorgeschlagen, bei denen die Funktionen "Stechen" zur Gewinnung von Kapillarblut und "Nachweisen" des Glucosegehalts als "Single-Test-Kit" verbraucherfreundlich vereint sind. Das am Stechort in situ gewonnene Blut muss dabei in der Regel über Kapillartransport gesammelt und mit einer Testchemie in Kontakt gebracht werden. Dabei soll der Einstichschmerz möglichst gering gehalten werden und eine hygienische Handhabung sichergestellt sein. Problematisch sind hierbei die Komplexität solcher Systems und der hohe Herstellungsaufwand.

Aus der WO 2005/001418 A ist ein Sammelsystem für Körperflüssigkeit bekannt, wofür auch eine mehrlagige Sterilbarriere vorgeschlagen wird, die im Zuge des Durchstechens an einer Schicht mit geringerer Festigkeit zunächst versagt, bevor das Stechelement eine darunterliegende erste Schicht durchdringt.

Ausgehend hiervon liegt der Erfindung die Aufgabe zugrunde, die im Stand der Technik aufgetretenen Nachteile zu vermeiden und ein gattungsgemäßes Produkt zu verbessern, so dass auch eine Massenfertigung mit günstigem Prozessablauf möglich ist.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Dementsprechend wird eine unter Vorspannung stehende und beim Anstechen eine Durchlassöffnung freigebende Sterilabdeckung für das Stechelement vorgeschlagen, wobei die lichte Weite der freigegebenen Durchlassöffnung größer als der Durchtrittsquerschnitt des Stechelements ist, so dass beim Zurückziehen kein Blut an der durchstochenen Folie abgestreift wird und eventuell das System kontaminiert. Dadurch ist eine Abschirmung zumindest von hygienisch relevanten Bereichen des Stechelements gegen das Eindringen von Keimen und anderen Verunreinigungen möglich. Die Vorspannung der Abdeckung kann zugleich genutzt werden, um unter Punktbelastung bzw. beim Anstechen durch das Stechorgan mit geringem Energieaufwand ähnlich wie bei einem platzenden Luftballon eine Durchlassöffnung schlagartig freizugeben. Damit wird eine ungewollte Abstumpfung des Stechorgans vermieden. Zudem wird so verhindert, dass hygienisch bedenkliche Partikel und Stanzreste beim Durchstoß der Sterilabdeckung entstehen. Umgekehrt wird auch die Gefahr einer Kontamination durch abgestreiftes Blut reduziert. Ein weiterer Vorteil liegt darin, dass eine einfache optische Kontrolle bei der Fertigung ausreicht, da auch kleine Fehler bzw. Löcher zu einem Versagen der Abdeckung führen. Die erfindungsgemäße Sterilbarriere lässt sich bei einfachen offenkanaligen Flachlanzetten, aber auch komplexeren Stechformteilen einsetzen.

Für eine Selbstöffnung beim Stechvorgang ist die Sterilabdeckung dem Stechorgan distal vorgeordnet, also vorderseitig dem Körper zugewandt.

Eine vorteilhafte Ausführung sieht vor, dass die Sterilabdeckung durch eine gummielastisch vorgespannte Aufreißmembran gebildet ist. Alternativ ist es auch möglich, dass die Sterilabdeckung durch eine unter Wärmeeinwirkung gespannte Schrumpfmembran gebildet ist.

Die Sterilabdeckung decht eine Öffnung eines Aufnahmeteils für das Stechelement ab. Auch herstellungstechnisch ist es von Vorteil, wenn das Stechelement in einem vorzugsweise als Spritzgussformteil ausgebildeten Aufnahmeteil angeordnet ist.

Der Stechvorgang wird vereinfacht, wenn das Aufnahmeteil eine in Stechrichtung verlaufende Schubführung für das Stechelement aufweist. Für eine selbsttätige Rückbewegung ist es vorteilhaft, wenn zwischen dem Aufnahmeteil und dem Stechelement eine beim Stechvorschub des Stechelements vorgespannte Rückstellfeder angeordnet ist.

Vorteilhafterweise besitzt das Aufnahmeteil Formschlussmittel zur formschlüssigen Halterung in einem Gehäuse.

Für ein integriertes System ist ein über den Kapillarkanal mit der Körperflüssigkeit beaufschlagbares Nachweiselement zum Nachweis eines Analyten in der Körperflüssigkeit vorgesehen.

Weitere Verbesserungen werden dadurch erreicht, dass das Nachweiselement ein quer zur Stechrichtung ausgedehntes Messfeld aufweist, und dass das Nachweiselement über Verbindungsmittel, insbesondere Rastmittel mit dem Stechelement gekoppelt ist und auf einer von dem Stechelement abgewandten Rückseite der flüssigkeitsdurchlässigen Sterilabdeckung angeordnet ist.

Vorteilhafterweise erfolgt dies dadurch, dass die Hohlkanüle relativ zu dem Stempel verschieblich ist, so dass Körperflüssigkeit in den Kanal eingesaugt und/oder daraus verdrängt wird. Eine weitere Verbesserung wird dadurch erreicht, dass der Stempel einen Querschnitt des Kanals vorzugsweise über eine umlaufende Dichtung abdichtet.

Um die Relativbewegung auf einfache Weise zu ermöglichen, ist es vorteilhaft, wenn der Stempel mit einem Aufnahmeteil für das Stechelement verschiebefest verbunden ist, wobei das Aufnahmeteil eine Schubführung für das Stechelement bildet.

Vorteilhafterweise werden die erfindungsgemäßen Testeinheiten so betrieben, dass das Stechelement durch einen Stechantrieb zumindest in einer Vorschubbewegung zum Einstechen in das Körperteil angetrieben ist. Dabei ist es für ein mikrofluidisches System ausreichend, wenn das Stechelement ein begrenztes Sammelvolumen für die Körperflüssigkeit von weniger als 100 Mikroliter, vorzugsweise weniger als 10 Mikroliter besitzt.

Im Folgenden wird die Erfindung anhand der in der Zeichnung schematisch dargestellten Ausführungsbeispiele näher erläutert. Es zeigen
- Fig. 1: ein Handgerät für Blutglucoseuntersuchungen mit einer darin eingesetzten Einweg-Testeinheit in teilweise geschnittener Ansicht;
- Fig. 2: die Testeinheit in einem gegenüber Fig. 1 vergrößerten Axialschnitt;
- Fig. 3a bis 3c: eine Draufsicht auf eine Sterilabdeckung der Testeinheit in verschiedenen Vorschubstellungen eines integrierten Stechelements;
- Fig. 4 und 5: weitere Ausführungsformen einer Testeinheit mit integriertem Nadelstempel; und
- Fig. 6: die Testeinheit nach Fig. 5 in verschiedenen Phasen der Flüssigkeitsgewinnung in geschnittener Teildarstellung.

Die in der Zeichnung dargestellte Testeinheit 10 lässt sich als disposibles Einwegteil in ein Handgerät 12 einsetzen, um mit integriertem Stechelement 14 und Nachweiselement 16 patientennahe Blutuntersuchungen für ein Glucosemonitoring zu ermöglichen.

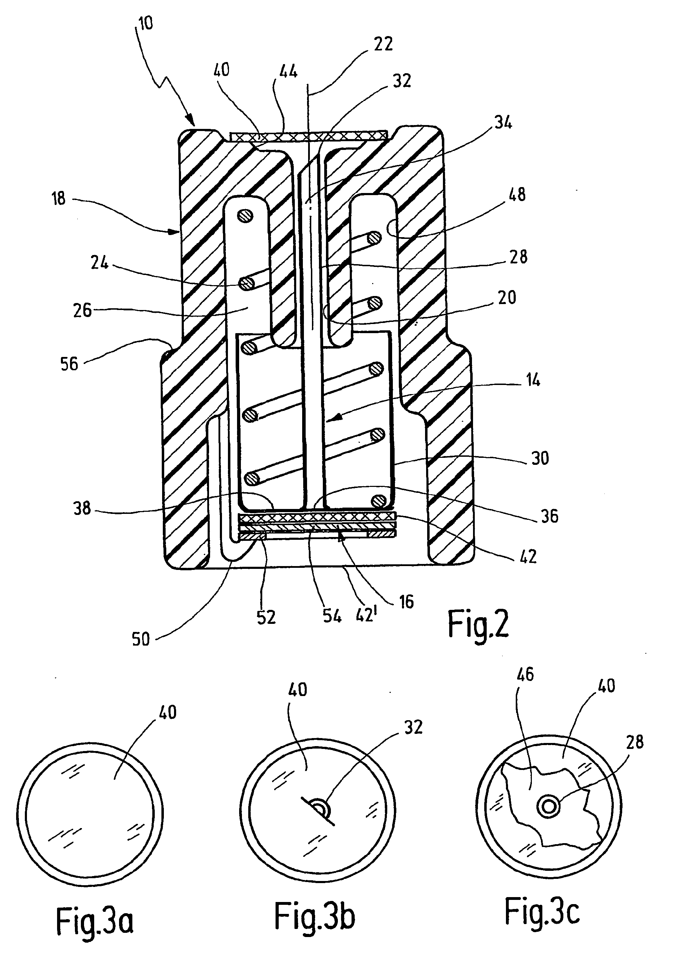

Wie am besten aus Fig. 2 ersichtlich, weist die Testeinheit 10 ein gehäuseartiges Aufnahmeteil 18 für das Stechelement 14 auf. Das Aufnahmeteil 18 bildet hierbei eine Schubführung 20, in der das Stechelement 14 in Richtung einer Stechachse 22 für eine hin- und hergehende Stechbewegung linear geführt ist. Die Rückbewegung erfolgt dabei unter der Kraft einer Rückstellfeder 24, die zwischen dem Stechelement 14 und dem Aufnahmeteil 18 in einem Federkäfig 26 vorgespannt ist.

Das Stechelement 14 ist durch ein einstückiges Tiefziehteil aus einem Edelstahlblech gebildet. Es umfasst eine Hohlkanüle 28 und eine proximal daran angeformte Flanschhülse 30. Mit ihrem angeschrägten distalen Ende bildet die Hohlkanüle 28 ein spitzes Stechorgan 32 zum Einstich in ein Körperteil eines Probanden. Das Innere der Hohlkanüle 28 dient als Kapillarkanal 34 für einen durch Kapillarkraft unterstützten Bluttransport von dem Stechorgan 32 zu einer proximalen Zentralmündung 36 an dem radial überstehenden - Stirnbund 38 der Flanschhülse 30.

Um eine sterildichte Abschirmung zumindest der Hohlkanüle 28 des Stechelements 14 gegen das Eindringen von Keimen und anderen Verunreinigungen zu ermöglichen, sind zwei Sterilabdeckungen 40, 42 vorgesehen. Die erste bzw. distale Sterilabdeckung 40 ist in Stech- bzw. Vorschubrichtung gesehen dem Stechorgan 32 vorgeordnet und überspannt einen freien Auslassquerschnitt 44 des Aufnahmeteils 18, während die zweite bzw. proximale Sterilabdeckung 42 die Kanalmündung 36 sterildicht überdeckt. Grundsätzlich ist es auch möglich, dass eine Sterilabdeckung 42' die proximale bzw. antriebsseitige Öffnung des Aufnahmeteils 18 abdeckt.

Zur Optimierung des Stechvorgangs auch in hygienischer Hinsicht ist die distale Sterilabdeckung 40 durch eine gummielastisch vorgespannte Aufreißmembran gebildet. Wie in Fig. 3 veranschaulicht, platzt diese Membran 40 beim Anstechen durch das Stechorgan 32 aufgrund der punktuellen Belastung schlagartig auf (Fig. 3b) und gibt eine große Durchlassöffnung 46 für den Durchtritt der Hohlkanüle 28 frei (Fig. 3c). Auf diese Weise kann eine ungewollte Körperkontamination mit Membranpartikeln und Stanzresten vermieden werden. Umgekehrt wird auch beim Zurückziehen der Kanüle 28 zuverlässig verhindert, dass Blut abgestreift wird und zu ungewollten Verunreinigungen führt.

Die proximale Sterilabdeckung 42 liegt flächig an den Stirnbund 38 der Flanschhülse 30 an. Dabei wird eine fluidische Verbindung des Kapillarkanals 34 mit dem Nachweiselement 16 durch ein Vliesmaterial ermöglicht. Dieses ist zumindest für Bestandteile, insbesondere Plasma der Blutflüssigkeit durchlässig, verhindert jedoch nach Art eines Sterilfilters den Durchtritt von Keimen. Zweckmäßig wird ein Faservlies für die Sterilabdeckung 42 verwendet. Für eine zusätzliche Keimabdichtung sollte die Flanschhülse 30 mit möglichst geringem Führungsspiel in der Bohrung 48 des Aufnahmeteils 18 gelagert sein. Daneben besteht auch die Option, das Stechelement 14 mit antibakteriell wirkenden Stoffen wie Silber oder Jod zu versehen.

Das Nachweiselement 16 ist auf der von dem Stechelement 14 abgewandten Rückseite der Sterilabdeckung 42 mittels eines Rasthakens 50 in flächiger Anlage gehalten. Zu diesem Zweck weist das Nachweiselement 16 einen Klemmring 52 auf, welcher auf ein scheibenförmiges Messfeld 54 randseitig aufgeklebt ist. Das Messfeld 54 kann ein Nachweisreagenz enthalten, welches auf Glukose in der zugeführten Blutflüssigkeit beispielsweise durch Farbänderung anspricht. Durch die beschriebene Halterung ist das Messfeld 54 orthogonal zur Stechachse 22 ausgerichtet, so dass eine reflektionsphotometrische Auswertung in Gerätelängsrichtung möglich ist.

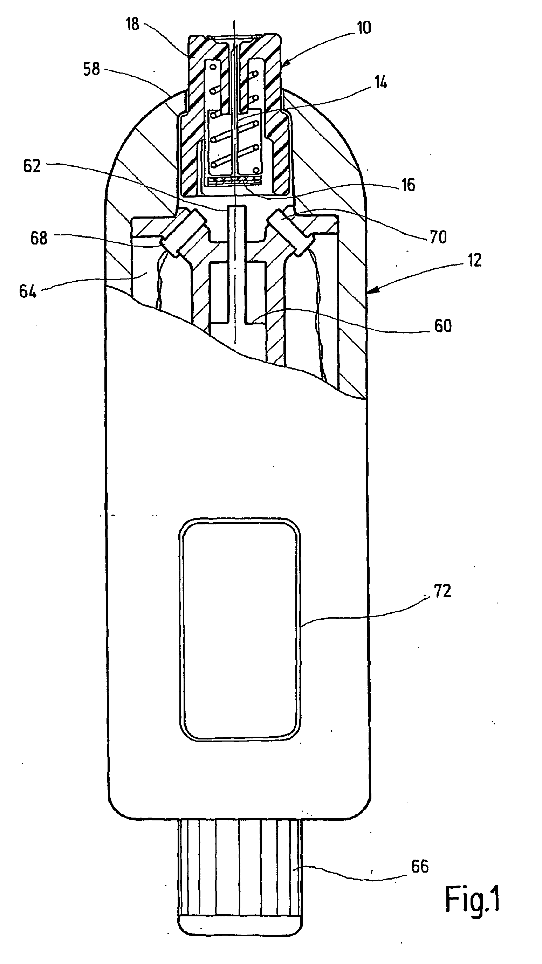

Das Aufnahmeteil 18 kann als Spritzguss-Formteil aus Kunststoff ausgebildet sein. Dabei ist es möglich, dass die distale Sterilabdeckung 40 ebenfalls aus dem Spritzgussmaterial besteht bzw. schon beim Spritzguss stirnseitig auf das Aufnahmeteil 18 aufgebracht wird. Denkbar ist es auch, dass die Rückstellfeder 24 aus Kunststoff mitgespritzt werden kann. Zur Zentrierung in dem Gerät 12 ist das Aufnahmeteil 18 mit einer Anschlagschulter 56 versehen, die mit einer komplementären Kontur des Gerätegehäuses 58 formflüssig in Eingriff bringbar ist, wie es in Fig. 1 gezeigt ist. Das Gerät 12 verfügt über einen Stechantrieb 60, dessen Stößel 62 über nicht gezeigte radiale Haltenoppen gegen den Klemmring 52 als Widerlager anstößt. Die Rückstellfeder 24 presst dabei den Stirnbund 38 des Stechelements 14 gegen das proximale Abdeckelement 42 und dieses gegen das Messfeld 54, so dass eine kapillare Kopplung für den Bluttransport sichergestellt ist.

Das Aufnahmeteil 18 kann über eine nicht gezeigte Dichtung luftdicht in das Gehäuse 58 eingespannt werden, wodurch der abgeschlossene Gehäuseinnenraum 64 auf Unterdruck gegenüber Atmosphäre bringbar ist. Dies lässt sich beispielsweise dadurch erreichen, dass der rückseitige Auslöser 66 des Stechantriebs 60 ähnlich einer Pipette konstruiert ist, um so bei Betätigung durch den entstehenden Unterdruck die Blutgewinnung und den Bluttransport in dem Kapillarkanal 28 zu unterstützen.

Nach dem Stechvorgang kann der Glucosenachweis berührungslos durch eine auf das Messfeld 54 ausgerichtete Photometeranordnung bestehend aus Lichtquelle 68 und Lichtempfänger 70 erfolgen. Das Messergebnis lässt sich sodann auf einer Anzeige 72 des Geräts 12 für den Benutzer anzeigen. Nach abgeschlossener Messung wird das Testelement 10 aus dem Gehäuse 58 entnommen und verworfen. Grundsätzlich ist es auch möglich, dass das Gerät 12 lediglich als Applikator mit Stechantrieb ausgebildet ist, während die Auswertung und gegebenenfalls die Magazinierung sowie Entsorgung der Testeinheiten 10 in einem gesonderten System vorgenommen wird.

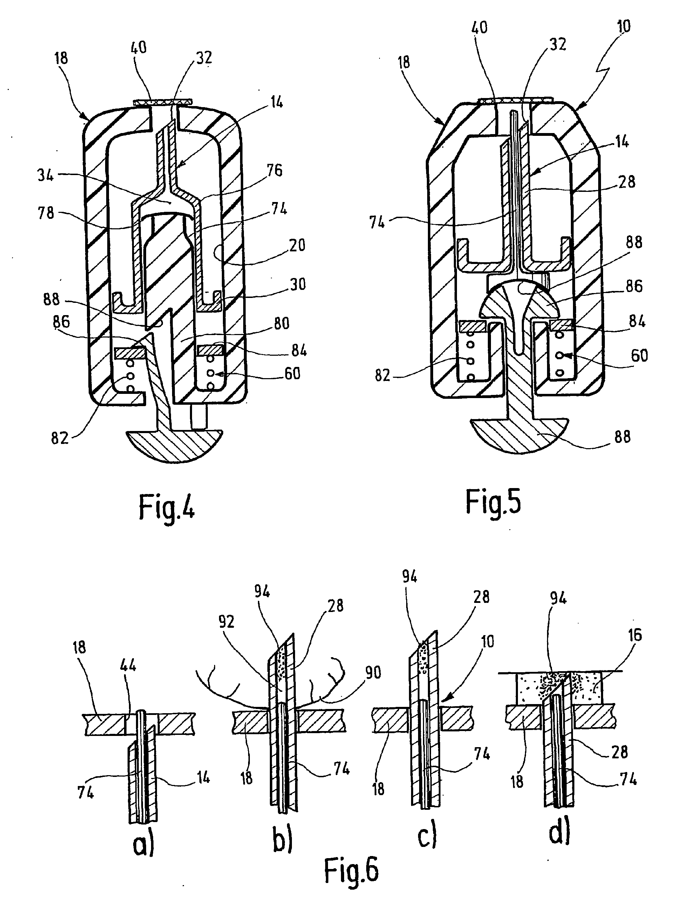

Bei den in Fig. 4 bis 6 dargestellten Ausführungsbeispielen sind gleiche Teile mit den gleichen Bezugszeichen wie vorstehend beschrieben versehen. Gemäß Fig. 4 ist ein Stempel 74 in einem erweiterten Abschnitt 76 des Stechelements 14 angeordnet. Dieser Stempel 74 ist über eine Dichtlippe 78 gegenüber der Wandung des Kanals 34 so abgedichtet, dass bei einer Relativbewegung des Stechelements 14 eine Druckveränderung zum Ansaugen bzw. Verdrängen von Blut auftritt. Zu diesem Zweck ist der Stempel 74 über ein Verbindungsteil 80 verschiebefest mit dem Aufnahmeteil 18 verbunden, während das Stechelement 14 in der Schubführung 20 linear beweglich gelagert ist. Der Stechantrieb 60 ist hier in Form einer vorgespannten Feder 82 in dem Aufnahmeteil 18 enthalten. Die Feder 82 ist in distaler Richtung an einem Haltering 84 abgestützt, welcher durch hakenförmige Spreizriegel 86 in seiner Ausgangsstellung gehalten wird. Bei Betätigung des Auslösers 88 gleiten die drei in Umfangsrichtung verteilten Riegel 86 in eine jeweilige Schräge 88 des Stempels 74 hinein, wodurch der Haltering 84 freigegeben wird und unter der Federwirkung das Stechelement 14 an seinem Bund 30 zum Einstechen in die Haut des Körperteils antreibt.

Bei der Ausführungsform nach Fig. 5 ist ein ähnlicher Auslösemechanismus 86,88 für den integrierten Federantrieb 60 des Stechelements 14 vorgesehen. Der Stempel 74 greift hier stiftförmig durch die gesamte Länge der Hohlkanüle 28 hindurch, wobei die bestehende feste Verbindung zu dem Aufnahmeteil 18 der Einfachkeit halber nicht eigens dargestellt ist.

Mittels eines solchen Testelements 10 kann eine Blutuntersuchung wie in Fig. 6 dargestellt ablaufen. In seiner Ausgangsstellung ist das Stechelement 14 mit darin befindlichem Stempel 74 in dem Aufnahmeteil 18 angeordnet (Fig. 6a). Der Benutzer setzt das Aufnahmeteil 18 im Bereich der Auslassöffnung 44 an ein Körperteil, insbesondere eine Fingerkuppe an und betätigt sodann den Auslöser 88. Dadurch wird die Hohlkanüle 28 in das Gewebe eingestochen, während der Stempel 74 ortsfest verbleibt und somit für einen Unterdruck in dem Aufnahmevolumen 92 sorgt (Fig. 6b). Das Aufnahmevolumen beträgt typischerweise wenige Mikroliter, so dass aufgrund der entsprechenden Nadeldimensionierung auch der Einstichschmerz und die Hautverletzung gering gehalten wird. Nach einer kurzen Verweilzeit hat sich genügend Blut 94 in dem Aufnahmevolumen 92 angesammelt, und die gesamte Anordnung 10 aus Aufnahmeteil 18 und Stechelement 14 kann unter Beibehaltung der Relativposition vom Benutzer aus dem Körperteil 90 herausgezogen und zu einer nicht eigens gezeigten Analyseeinheit transferiert werden (Fig. 6c). Die Analyseeinheit umfasst ein Test- bzw. Nachweiselement 16 mit einer auf den Analyten (Glukose) in der Blutprobe 94 ansprechenden Testchemie. Wie in Fig. 6d gezeigt, drückt der Benutzer die Hohlkanüle 28 mit ihrem distalen Ende gegen das Nachweiselement 16 an, wodurch unter Kompression der Feder 82 eine Rückbewegung auf dem Sternpel 74 für eine entsprechende Verdrängung der aufgenommenen Blutmenge sorgt. Anschließend kann das Testelement 10 entsorgt werden, während die Analyse automatisch durchgeführt wird.

Bei der Herstellung der Testeinheiten als Massenprodukt mit integriertem Sammel- und Nachweissystem lässt sich die erforderliche Sterilisierung des Stechelements in Gegenwart von strahlungsempfindlichen Testreagenzien auf dem Nachweiselement nur schwer realisieren. Eine verfahrensmäßige Lösung sieht daher einen mehrstufigen Arbeitsablauf vor. Zunächst wird in insteriler Umgebung das Stechelement 14 in dem Aufnahmeteil vormontiert, wobei zumindest der Kapillarkanal 34 durch die Sterilabdeckungen 40, 42 isoliert wird. Sodann erfolgt eine Gammasterilisation, wobei das Stechelement bzw. Tiefziehteil 14 steril eingeschlossen bleibt. Anschließend kann das Nachweiselement 16 insteril eingesetzt werden, so dass insgesamt auf Sterilbedingungen bei der Prozessführung verzichtet werden kann.

## Patentansprüche

1. Testeinheit für Einmaluntersuchungen einer Körperflüssigkeit, insbesondere Blut, mit einem in ein Körperteil einstechbaren Stechelement (14), das einen von einem Stechorgan (32) zu einer Zielstelle (36) führenden Kapillarkanal (34) für den Transport der Körperflüssigkeit aufweist, und mit einer Sterilabdekung (40) für das Steckelement (14) die eine Öffnung eines Aufnahmeteils (18) für das Steckelement (14) abdeckt und dem Steckorgan (32) distal vorgeordnet ist, **dadurch gekennzeichnet, daß** die Sterilabdeckung (40) unter Vorspannung steht und beim Anstechen eine Durchlassöffnung (46) freigibt,
wobei die lichte Weite der freigegebenen Durchlassöffnung (46) größer als der Durchtrittsquerschnitt des Stechelements (14) ist.

2. Testeinheit nach Anspruch 1, **dadurch gekennzeichnet, dass** die Sterilabdeckung (40) durch eine gummielastisch vorgespannte Aufreißmembran gebildet ist.

3. Testeinheit nach Anspruch 1, **dadurch gekennzeichnet, dass** die Sterilabdeckung (40) durch eine unter Wärmeeinwirkung gespannte Schrumpfmembran gebildet ist.

4. Testeinheit nach einem der Ansprüche 1 bis 3, **dadurch gekennzeichnet, dass** die lichte Weite der freigegebenen Durchlassöffnung (46) ein Mehrfaches größer als der Durchtrittsquerschnitt des Stechelements (14) ist.

5. Testeinheit nach einem der Ansprüche 1 bis 4, **dadurch gekennzeichnet, dass** das Stechelement (14) in einem vorzugsweise als Spritzgussformteil ausgebildeten Aufnahmeteil (18) angeordnet ist.

6. Testeinheit nach Anspruch 5, **dadurch gekennzeichnet, dass** das Aufnahmeteil (18) eine in Stechrichtung verlaufende Schubführung (20) für das Stechelement (14) aufweist.

7. Testeinheit nach Anspruch 5 oder 6, **dadurch gekennzeichnet, dass** zwischen dem Aufnahmeteil (18) und dem Stechelement (14) eine beim Stechvorschub des Stechelements (14) vorgespannte Rückstellfeder (24) angeordnet ist.

8. Testeinheit nach einem der Ansprüche 5 bis 7, **dadurch gekennzeichnet, dass** das Aufnahmeteil (18) Formschlussmittel (56) zur formschlüssigen Halterung in einem Gehäuse (58) aufweist.

9. Testeinheit nach einem der Ansprüche 1 bis 8, **gekennzeichnet durch** ein vorzugsweise über den Kapillarkanal (34) mit der Körperflüssigkeit beaufschlagbares Nachweiselement (16) zum Nachweis eines Analyten in der Körperflüssigkeit.

10. Testeinheit nach Anspruch 9, **dadurch gekennzeichnet, dass** das Nachweiselement (16) ein quer zur Stechrichtung ausgedehntes Messfeld (54) aufweist.

11. Testeinheit nach Anspruch 9 oder 10, **dadurch gekennzeichnet, dass** das Nachweiselement (16) über Verbindungsmittel (50), insbesondere Rastmittel mit dem Stechelement (14) gekoppelt ist.

12. Testeinheit nach einem der Ansprüche 1 bis 11, **dadurch gekennzeichnet, dass** in dem als Hohlkanüle ausgebildeten Stechelement (14) ein Stempel (74) angeordnet ist.

13. Testeinheit nach Anspruch 12, **dadurch gekennzeichnet, dass** die Hohlkanüle (28) relativ zu dem Stempel (74) verschieblich ist, so dass Körperflüssigkeit in den Kanal (34) eingesaugt und/oder daraus verdrängt wird.

14. Testeinheit nach Anspruch 12 der 13, **dadurch gekennzeichnet, dass** der Stempel (74) einen Querschnitt des Kanals (34) vorzugsweise über eine umlaufende Dichtung (78) abdichtet.

15. Testeinheit nach einem der Ansprüche 12 bis 14, **dadurch gekennzeichnet, dass** der Stempel (74) mit einem Aufnahmeteil (18) für das Stechelement (14) verschiebefest verbunden ist, wobei das Aufnahmeteil eine Schubführung für das Stechelement (14) bildet.

16. Testeinheit nach einem der Ansprüche 1 bis 15, **dadurch gekennzeichnet, dass** das Stechelement (14) durch einen Stechantrieb (60) zumindest in einer Vorschubbewegung zum Einstechen in das Körperteil angetrieben ist.

17. Testeinheit nach einem der Ansprüche 1 bis 16, **dadurch gekennzeichnet, dass** das Stechelement (14) ein begrenztes Sammelvolumen für die Körperflüssigkeit von weniger als 100 Mikroliter, vorzugsweise weniger als 10 Mikroliter besitzt.

## Claims

1. Test unit for carrying out one-time analyses in a body fluid, in particular blood, comprising a lancing element (14) which can be pricked into a body part and has a capillary channel (34) that leads from a lancing member (32) to a target site (36) for transporting the body fluid, and a sterile cover (40) for the lancing element (14) which covers an opening of a receiving part (18) for the lancing element (14) and is arranged distally in front of the lancing member (32), **characterized in that** the sterile cover (40) is pretensioned and when pierced uncovers a passage opening (46), wherein the clear width of the uncovered passage opening (46) is larger than the piercing cross-section of the lancing element (14).

2. Test unit according to claim 1, **characterized in that** the sterile cover (40) is formed by a rubber-elastic pretensioned tear-membrane.

3. Test unit according to claim 1, **characterized in that** the sterile cover (40) is formed by a shrink-membrane that is tensioned by the action of heat.

4. Test unit according to one of the claims 1 to 3, **characterized in that** the clear width of the uncovered passage opening (46) is several times larger than the piercing cross-section of the lancing element (14).

5. Test unit according to one of the claims 1 to 4, **characterized in that** the lancing element (14) is arranged in a receiving part (18) preferably designed as an injection-moulded part.

6. Test unit according to claim 5, **characterized in that** the receiving part (18) has a push guide (20) for the lancing element (14) running in the lancing direction.

7. Test unit according to claims 5 or 6, **characterized in that** a return spring (24) that is pretensioned during the lancing advance movement of the lancing element (14) is arranged between the receiving part (18) and the lancing element (14).

8. Test unit according to one of the claims 5 to 7, **characterized in that** the receiving part (18) has form-locking means (56) to hold it in a form-locking manner in a housing (58).

9. Test unit according to one of the claims 1 to 8, **characterized by** a detection element (16) for detecting an analyte in the body fluid to which body fluid can be applied preferably via the capillary channel (34).

10. Test unit according to claim 9, **characterized in that** the detection element (16) has a measuring field (54) extending transversely to the lancing direction.

11. Test unit according to claims 9 or 10, **characterized in that** the detection element (16) is coupled with the lancing element (14) by connecting means (50) and in particular latching means.

12. Test unit according to one of the claims 1 to 11, **characterized in that** a plunger (74) is arranged in the lancing element (14) designed as a hollow cannula.

13. Test unit according to claims 12, **characterized in that** the hollow cannula (28) can be moved relative to the plunger (74) so that body fluid is sucked into the channel (34) and/or displaced therefrom.

14. Test unit according to claim 12 or 13, **characterized in that** the plunger (74) seals a cross-section of the channel (34) preferably by means of a circumferential seal (78).

15. Test unit according to one of the claims 12 to 14, **characterized in that** the plunger (74) is connected with a receiving part (18) for the lancing element (14)in a non-displaceable manner wherein the receiving part forms a push guide for the lancing element (14).

16. Test unit according to one of the claims 1 to 15, **characterized in that** the lancing element (14) is driven by a lancing drive (60) at least in an advance movement for pricking into the body part.

17. Test unit according to one of the claims 1 to 16, **characterized in that** the lancing element (14) has a limited collection volume for body fluid of less than 100 microliters, preferably of less than 10 microliters.

## Revendications

1. Unité d'analyse à usage unique d'un liquide corporel, en particulier le sang, comportant un élément de piqûre (14) qui peut être introduit dans une partie du corps, lequel élément présente un conduit capillaire (34) qui mène d'un organe de piqûre (32) à un point cible (36) pour le transport du liquide corporel, et comportant un élément de recouvrement stérile (40) pour l'élément de piqûre (14), qui recouvre une ouverture d'une pièce de réception (18) pour l'élément de piqûre (14) et est disposé de manière distale en amont de l'organe de piqûre (32), **caractérisée en ce que** l'élément de recouvrement stérile (40) est précontraint et libère une ouverture de passage (46) lors de la piqûre, le diamètre intérieur de l'ouverture de passage (46) libérée étant supérieur à la section transversale de passage de l'élément de piqûre (14).

2. Unité d'analyse selon la revendication 1, **caractérisée en ce que** l'élément de recouvrement stérile (40) est formé par une membrane pouvant être déchirée, prétendue de manière élastique.

3. Unité d'analyse selon la revendication 1, **caractérisée en ce que** l'élément de recouvrement stérile (40) est formé par une membrane rétractable tendue sous l'effet de la chaleur.

4. Unité d'analyse selon l'une des revendications 1 à 3, **caractérisée en ce que** le diamètre intérieur de l'ouverture de passage (46) libérée est plusieurs fois plus grand que la section transversale de passage de l'élément de piqûre (14).

5. Unité d'analyse selon l'une des revendications 1 à 4, **caractérisée en ce que** l'élément de piqûre (14) est disposé dans une pièce de réception (18) configurée de préférence en tant que pièce moulée par injection.

6. Unité d'analyse selon la revendication 5, **caractérisée en ce que** la pièce de réception (18) présente un guidage de poussée (20) se déplaçant dans le sens de la piqûre pour l'élément de piqûre (14).

7. Unité d'analyse selon la revendication 5 ou 6, **caractérisée en ce qu'**entre la pièce de réception (18) et l'élément de piqûre (14) est disposé un ressort de rappel (24) prétendu lors de la poussée de l'élément de piqûre (14) en vue de la piqûre.

8. Unité d'analyse selon l'une des revendications 5 à 7, **caractérisée en ce que** la pièce de réception (18) présente des moyens de complémentarité de forme (56) pour le maintien par complémentarité de forme dans un logement (58).

9. Unité d'analyse selon l'une des revendications 1 à 8, **caractérisée par** un élément de détection (16) pouvant être alimenté de préférence par le biais du conduit capillaire (34) avec le liquide corporel pour la détection d'un analyte dans le liquide corporel.

10. Unité d'analyse selon la revendication 9, **caractérisée en ce que** l'élément de détection (16) présente une zone de mesure (54) prolongée transversalement au sens de la piqûre.

11. Unité d'analyse selon la revendication 9 ou 10, **caractérisée en ce que** l'élément de détection (16) est couplé à l'élément de piqûre (14) par le biais de moyens de liaison (50), en particulier des moyens d'encliquetage.

12. Unité d'analyse selon l'une des revendications 1 à 11, **caractérisée en ce qu'**un poinçon (74) est disposé dans l'élément de piqûre (14) configuré en tant que canule creuse.

13. Unité d'analyse selon la revendication 12, **caractérisée en ce que** la canule creuse (28) est déplaçable par rapport au poinçon (74), de sorte que le liquide corporel est aspiré dans le conduit (34) et/ou en est chassé.

14. Unité d'analyse selon la revendication 12 ou 13, **caractérisée en ce que** le poinçon (74) bouche une section transversale du conduit (34) de préférence par le biais d'un joint circulaire (78).

15. Unité d'analyse selon l'une des revendications 12 à 14, **caractérisée en ce que** le poinçon (74) est relié de manière non déplaçable à une pièce de réception (18) pour l'élément de piqûre (14), la pièce de réception formant un guidage de poussée pour l'élément de piqûre (14).

16. Unité d'analyse selon l'une des revendications 1 à 15, **caractérisée en ce que** l'élément de piqûre (14) est entraîné par le biais d'un entraînement de piqûre (60) au moins dans un mouvement de poussée pour réaliser une piqûre dans la partie du corps.

17. Unité d'analyse selon l'une des revendications 1 à 16, **caractérisée en ce que** l'élément de piqûre (14) possède un volume collecteur limité pour le liquide corporel de moins de 100 microlitres, de préférence de moins de 10 microlitres.
